(19) Europäisches Patentamt European Patent Office Office européen des brevets

(11) **EP 2 409 638 A1**

(12) **EUROPEAN PATENT APPLICATION**
published in accordance with Art. 153(4) EPC

(43) Date of publication:
**25.01.2012 Bulletin 2012/04**

(21) Application number: **10753582.5**

(22) Date of filing: **18.03.2010**

(51) Int Cl.:
*A61B 5/00* (2006.01)      *A61B 5/11* (2006.01)

(86) International application number:
**PCT/JP2010/054701**

(87) International publication number:
**WO 2010/107091 (23.09.2010 Gazette 2010/38)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **18.03.2009 FR 0951714**

(71) Applicant: **Aisin Seiki Kabushiki Kaisha Aichi 448-8650 (JP)**

(72) Inventors:
• **VRAZIC, Sacha**
  **Kariya-shi**
  **Aichi 448-8650 (JP)**
• **BOBILLET, William**
  **Kariya-shi**
  **Aichi 448-8650 (JP)**

(74) Representative: **Kuhnen & Wacker
Patent- und Rechtsanwaltsbüro
Prinz-Ludwig-Strasse 40A
85354 Freising (DE)**

(54) **BIOLOGICAL PARAMETER MONITORING METHOD, COMPUTER PROGRAM, AND BIOLOGICAL PARAMETER MONITORING DEVICE**

(57)    A method for monitoring a biological parameter out of either the heartbeat and/or respiratory signal of an occupant (2) on a member of a seat (8) or bed, wherein a signal or signals received from one or a plurality of sensors (6) connected to the member and capable of detecting the variation of pressure due to contact are processed by non-linear filtering. For example, the method is mounted on a vehicle and used. Also provided is a computer program including code instructions capable of controlling execution of the method of the invention when the method is executed by a computer. Further provided is a monitoring device for monitoring a biological parameter out of either the heartbeat and/or respiratory signal of an occupant (2) on a member of a seat or bed.

## FIG. 1

EP 2 409 638 A1

**Description**

Technical Field

The present invention relates to monitoring a biological parameter of an occupant on a member of a seat or a bed.

Background Art

The present invention particularly relates to extracting and monitoring a biological parameter of an occupant such as a driver or a passenger in a vehicle, but is not limited thereto. The present invention is particularly intended to extract a heartbeat and respiratory signal of a human without restraining the human and, if possible, under all driving conditions. By obtaining these biological parameters, a monitoring system can be used for improving a road traffic safety. It is especially desired to reduce the number of traffic accidents due to drowsiness or symptoms of illness.

For example, Patent Literature 1 discloses a method for monitoring a heartbeat of an occupant on a seat. For this purpose, the seat has piezoelectric sensors, and by analyzing signals from the piezoelectric sensors, a heartbeat signal is extracted. However, the signals transmitted from the respective sensors contain noise that is unrelated to a desired biological signal. The aforementioned literature discloses that in order to remove this noise, a passband filter is used, and only a frequency range containing a frequency of a phenomenon to be studied in signals is taken into consideration.

However, practically, this method does not produce a satisfactory result. This is because, practically, noise in the signals from the sensors has all frequency ranges. Consequently, the passband filter cannot isolate a desired signal by removing this noise. In other words, even if the passband filter limits the frequency to a certain frequency range, a desired signal cannot be obtained due to a noise amount remaining in this range. In addition, if such a method is desirably mounted on a vehicle and used in order to monitor a biological parameter of a driver or passenger, a satisfactory result cannot be obtained due to a noise level caused by vehicle vibration.

Patent Literature 1: US Patent Application Publication No.2008/0103702

Disclosure of Invention

Problem to be Solved by the Invention

Accordingly, an object of the present invention is to improve estimation of a biological signal in an environment with strong noise.

Means to Solve the Problem

To achieve the object, the present invention provides a method for monitoring at least one biological parameter out of a heartbeat and/or a respiratory signal of an occupant on a member of a seat or a bed, the method characterized by comprising the step of processing a signal or respective signals by non-linear filtering, received from one or more sensors connected to the member and capable of detecting a change of pressure due to contact.

Use of non-linear filtering produces a good result as long as a phenomenon to be observed acts as a nonlinear system. Therefore, a parameter can be estimated in collected signals in the presence of noise by non-linear filtering, and therefore a signal regarding a parameter to be monitored can be easily extracted.

Advantageously, filtering includes a Bayesian recursive estimator such as an extended Kalman filter or an individual filter.

Use of a Bayesian tool is effective especially when a value such as a frequency that cannot be directly observed is estimated in the aforementioned state. In the case where the extended Kalman filter is used in the presence of a nonlinear system, especially if a nonlinear shape can be modeled, a good result can be obtained. Similarly, for better identifying a change of noise other than gaussian noise, it turns out that the individual filter is especially suitable.

Advantageously, a linear state transition equation of a following type is used:

$$x_{k+1} = A \cdot x_k + v_k$$

where $x_{k+1}$ is a vector representing a state of a following state, and

[Expression 1]

$$\hat{\mathbf{x}}_k = \begin{bmatrix} \omega_k \\ a_{k,1} \\ \vdots \\ a_{k,m} \\ \phi_{k,1} \\ \vdots \\ \phi_{k,m} \end{bmatrix}, \quad \mathbf{A} = \begin{bmatrix} 1 & & & & & & \\ & 1 & & & & & \\ & & \ddots & & & & \\ & & & 1 & & & \\ 1 & & & & 1 & & \\ \vdots & & & & & \ddots & \\ m & & & & & & 1 \end{bmatrix}$$

$v_k$ is white gaussian noise.

Advantageously, an output signal from a sensor or one of a plurality of sensors is modeled in the following manner.

[Expression 2]

$$y(t) = \sum_{i=1}^{m} a_i(t) \cdot \sin \phi_i(t)$$

where

$$\varphi_1(t) = \omega(t) \cdot t$$

$$\varphi_i(t) = i \cdot \omega(t) \cdot t + \theta_i(t), \ i = 2...m,$$

$y(t)$ represents a signal,
$\omega(t)$ represents a momentary basic pulse of the signal,
m represents the number of sine functions,
$a_i(t)$ represents an amplitude of a sine function,
$\varphi_i(t)$ represents a momentary phase of a higher harmonic wave, and

$\theta_i(t)$ represents a phase difference between the basic pulse and the higher harmonic wave.

Advantageously, the following observation expression is used.

[Expression 3]

$$\hat{\mathbf{y}}_k^- = \mathbf{H}(\hat{\mathbf{x}}_k^-, \mathbf{w}_k) = \sum_{i=1}^{m} \hat{a}_{i,k} \cdot \sin \hat{\phi}_{i,k} + \mathbf{n}_k$$

where

[Expression 4]

$$\hat{\mathbf{y}}_k^-$$

represents an estimation value of a signal y(k) that is estimated by an observer, H represents a matrix associating a state $x_k$ with a measured value $y_k$,

[Expression 5]

$$\hat{a}_{i,k}$$

and

[Expression 6]

$$\hat{\phi}_{i,k}$$

represent estimation values of $a_{i,k}$ and $\varphi_{i,k}$ that are estimated by the observer, respectively, and $n_k$ represents noise observed by the observer.

It is preferable
to receive a signal from each of a group of sensors,
to input this signal to an atom dictionary (dictionnaire d'atomes in French),
to select several sensors on a basis of the input, and
to use only the selected sensors to perform monitoring.

In this way, by inputting a signal to the atom dictionary, a sensor to supply the most suitable signal for monitoring a biological parameter can be selected by an automated means with high reliability. For this reason, this monitoring system can be adapted in real time to the physical features and posture of the occupant of a member, a change of the posture, or a movement of the occupant. Therefore, according to the present method, a biological parameter can be monitored by spatially and temporally adapting to various conditions for monitoring at the same time, without intervention of an operator. Consequently, the present method facilitates obtaining the most reliable data regarding a biological parameter to be monitored.

Moreover, when the present method is mounted on a vehicle, for example, a vibration level is configured such that most of reliability of the present method depends on a type of processing performed in a previous stage to consider ambient noise. In other words, by simply removing most of the noise from a signal to be utilized in the previous stage, data indicating a biological parameter to be monitored is suitably extracted in most cases. This problem is not limited to monitoring when the present method is mounted and used on a vehicle. In the case where monitoring relates to, for example, a patient occupying a bed, since ambient noise (for example, noise caused by an electric appliance) may disturb this monitoring, as a result, reliability of the present method similarly depends on quality of processing performed for removing the noise from a signal to be utilized. In a medical bed, when the bed moves, vibration occurs, and therefore this problem occurs when a patient is carried.

To solve this problem, it is preferable

to connect at least one accelerometer to the member,

to decide a model of a transfer function between at least one signal in input from the one accelerometer out of the at least one accelerometer and a signal in output from one sensor of one or more sensors.

to estimate a noise value with use of the model, and

to remove the estimated noise value from the signal from the sensor.

In this way, this accelerometer or these accelerometers detect(s) vibration by definition, thereby supplying a high fidelity reference signal of ambient noise. Moreover, by deciding the model of the transfer function, a noise value can be estimated with high reliability. Accordingly, from a signal to be utilized, most of the noise contained in this signal can be removed. This does not mean directly removing signals read by this accelerometer or these accelerometers from signals to be utilized, that is, this does not mean subtraction of signals. In other words, by using a signal from the accelerometer to model an effect of noise in a signal to be utilized, an estimation value of this noise is more suitably extracted from the signal to be utilized. By this, the signal to be utilized without most noise can be obtained in the previous stage. The remaining noise does not obstruct obtaining a signal to be utilized faithfully indicating one or more biological parameters to be utilized.

The present method is used mounted on a vehicle, for example.

The present invention also provides a computer program including a code instruction, the code instruction being able to control performance of a method according to the present invention when the method is performed by a computer or a calculator.

The present invention also provides a data recording medium including such a program in a recorded form.

The present invention further provides such a program in such a way that it can be used by download in a remote communication network.

Moreover, the present invention provides

a device for monitoring at least one biological parameter out of a heartbeat and/or a respiratory signal of an occupant on a member of a seat or a bed, the device including:

at least one sensor being connected to the member and being able to detect a change of pressure due to contact; and a means to process a signal or respective signals received from the sensor or respective sensors by non-linear filtering.

Other features and advantages of the present invention will be apparent from description of preferred embodiments with reference to drawings, not for limitation but for examples.


Brief Description of Drawings

Fig. 1 is an overall view illustrating a device according to an embodiment of the present invention.
Fig. 2 is a side view of a seat connected to the present device.
Fig. 3 is a view illustrating one of sensor arrays of the seat in Fig. 2.
Fig. 4 is a graph indicating an atom of a dictionary used in a method according to the present invention.
Fig. 5 is a diagram illustrating modeling a transfer function according to the present embodiment.
Fig. 6 is a diagram illustrating that such a modeled transfer function is taken into consideration in performing the present method.
Fig. 7 is a graph illustrating an amplitude of a signal before and after extraction of noise by a transfer function over time.

Fig. 8 is a graph of two curves, one indicating a heartbeat estimated by the present method, and the other one indicating a real heartbeat, and also indicates an acceptable range.

Best Mode for Carrying Out the Invention

A method for configuring a complete system to extract biological information from the body of a human 2 who is a passenger or driver on a vehicle 4 and a device therefor according to embodiments of the present invention will be described below.
The present invention is intended to monitor a biological parameter of a human such as a heartbeat and/or a respiratory rhythm. This monitoring is required to be performed under various driving conditions and be accurate according to a physical movement.
The present invention relates to obtaining and monitoring the aforementioned biological parameter. Especially, it is desired that the aforementioned parameter can be obtained under any driving condition without restraining a person of which the parameter is to be obtained. Actually, by obtaining information on a heartbeat and/or a respiratory signal, a monitoring system can be obtained to reduce the number of accidents due to drowsiness or several symptoms of illness, thereby improving a road traffic safety.

First, a configuration of the system and steps performed in the present method will be generally described. Then, such a configuration and several features of the present embodiment will be described in detail.

A device includes a plurality of piezoelectric sensors 6 supported by a seat 8 occupied by the human 2. These sensors are configured so as to always obtain desired signals regardless of the posture or movement of the occupant. The sensors can detect a change of a contact pressure, and in this device, the piezoelectric sensors are employed. The sensors are placed on a seating portion 10 of the seat 8 and in the vicinity of an upper portion of a front face of a seat back 12, and the front face is provided so as to be adjacent to the occupant 2. The sensors can also be directly placed on this face. In this way, the sensors receive a pressure by the body especially in the vicinity of an artery of the occupant 2 and a change of pressure by the body. In this case, the sensors include a film or a sheet. However, as will be described later, since these sensors detect all types of mechanical vibrations, the desired signals cannot be directly observed from outputs of such sensors.

In this embodiment, the seat 8 includes about 60 sensors, but it should be appreciated that the number of the sensors is not limited to this. The number of sensors placed on the seating portion 10 is 10 to 70, and may be 40, for example. The number of sensors on the seat back 12 is 5 to 50, and may be 20, for example. A substrate used during the use of the present method includes, for example, 20 sensors, that is, 15 on the seating portion and 5 on the seat back. Accordingly, the substrate includes about 30% of the sensors placed in the seat.

Signal processing for the sensors uses an analog process and a digital process.

The analog process includes the steps of collecting and organizing amplitudes of output signals from the sensors 6. Each of the analog outputs is digitalized, and then several sensors are automatically selected according to a position of the body of the occupant 2 on the seat 8. Signals transmitted from these sensors are subsequently processed and combined. Such steps of selecting and combining on the sensors are performed suitably according to movement of the body and so as to predict the movement at a plurality of number of times during performing the present method. It should be noted that movement of a passenger and movement of a driver in a vehicle are generally different. The movement of the passenger is more random than that of the driver, but the number of the movement is less than that of the driver. As for the movement of the driver, characteristics such as four drive wheels in the vehicle, movement of hands of the driver, and a position of feet for acceleration, braking or changing gear must be taken into consideration.

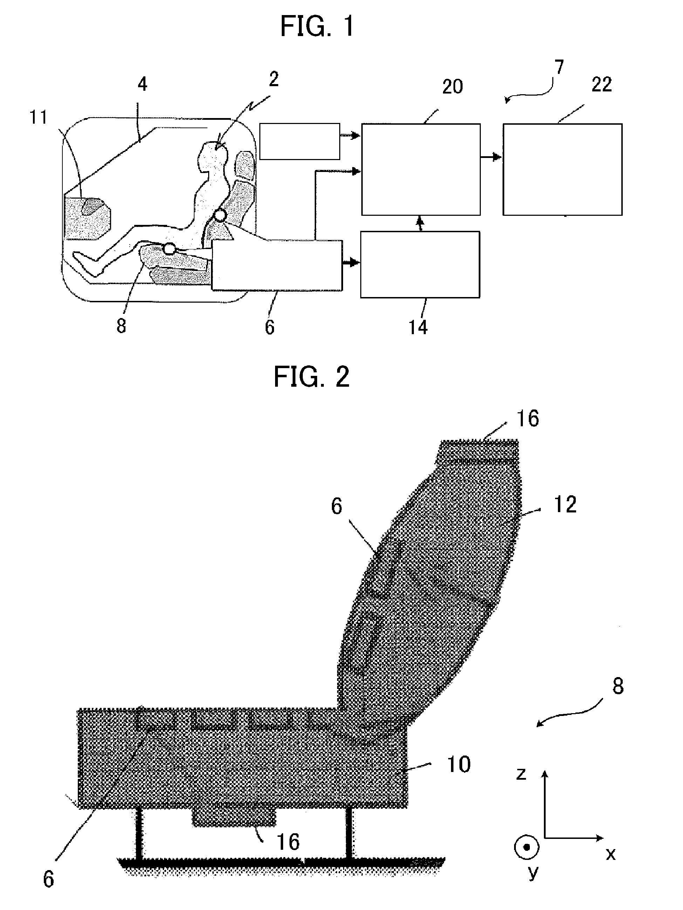

In Fig. 1, a unit that selects and combines signals from the sensors is illustrated as a block 14. This block can detect the movement of the body to track and predict it. Especially, the block can select a sensor that is most able to supply an effective signal in order to obtain a biological parameter. The block can accurately detect one movement, predict one movement and obtain a list of candidate sensors to be selected. Accordingly, the present method is continuously performed regardless of movement of the occupant. The block can also determine whether only an inertial object is placed on the member, and if placed, can be adapted not to perform any processing.

The seat 8 is further provided with accelerometers 16 that work as reference sensors for ambient noise such as vibration noise caused especially by the vehicle. These accelerometer sensors 16 detect noise in three directions that are vertical to one another, that is, horizontal directions X, Y and vertical direction Z, respectively. In the scope of the present method, a transfer function between the accelerometers 16 and the respective piezoelectric sensors 6 is estimated, and after that, output noise from these sensors can be reduced. The transfer function is re-estimated whenever re-estimation is determined to be necessary for tracking current driving conditions of the vehicle and movement of the body of the occupant. A block 20 plays a role of generating a dynamic model that is changing over time in order to estimate the transfer function between the accelerometers and piezoelectric sensors. With the use of the transfer function estimated as above, various values regarding noise are predicted, and after that, noise transferred by signals from piezoelectric sensors can be reduced. The model used in this stage may be linear or non-linear. This is a first step of reducing noise of signals supplied by the sensors 6.

Meanwhile, this step of reducing noise may be determined to be insufficient. Therefore, the block 20 further includes a second estimation and tracking step for obtaining a biological parameter such as a heartbeat and/or a respiratory signal more accurately. In this step, a change of the biological parameter can be tracked more accurately regardless of driving conditions (movement of the body of the occupant, driving in cities, high-speed driving and driving on an express way). This second step is configured to adapt to a non-linear system. This step can include the extended Kalman filter or individual filter so as to better identify all noise variations. Such a non-linear processing step is highly suitable for extracting and monitoring a parameter deriving from a non-linear model, such as a heartbeat and respiratory rhythm in a vibrating environment changing over time.

After the two filtering steps, a block 22 can obtain and monitor a desired signal and can predict a change of the signal.

The present invention is suitable for all types of vehicles. However, the present invention is not limited to vehicles, and can be applied to other types of members such as a seat or bed, for example, a medical bed for monitoring a parameter of a patient.

A method to be used is self-adapting.

Next, several features of the present method will be described in detail. It is assumed here that a biological parameter of a driver of a vehicle is monitored.

1. Selecting and combining piezoelectric sensors

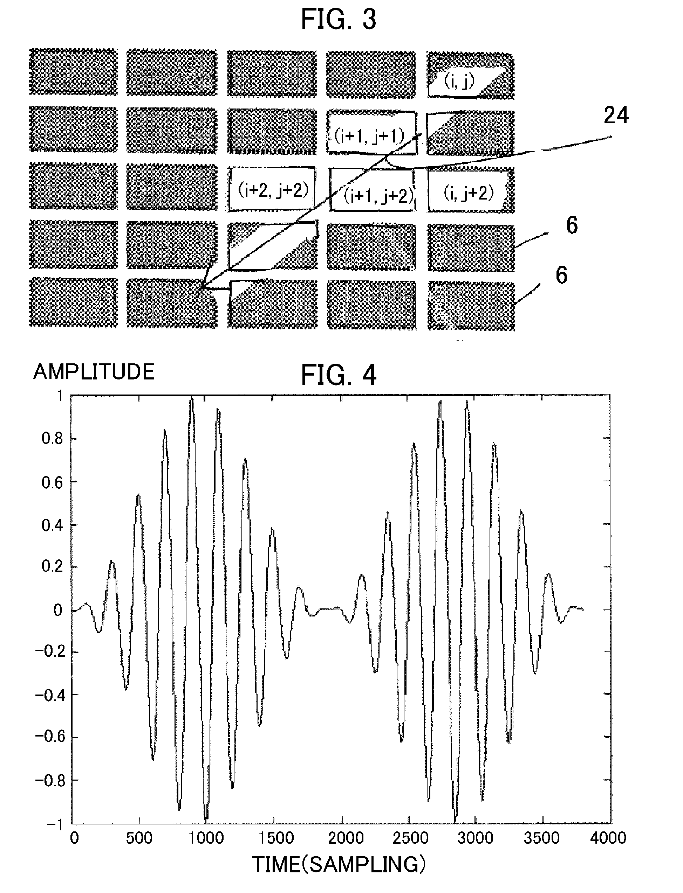

Referring to Figs. 1 to 3, the seat 8 has two sensor arrays 6, one being placed in the seating portion 10 and the other being placed in seat back 12. Each of these arrays includes a plurality of rows and a plurality of columns in this case. For example, as illustrated in Fig. 3, the array includes five rows and five columns, and forms grid-like sensors. Sensors of each array are arranged on approximately the same plane. The sensors are electrically connected to other portions of the device in a suitable manner, and signals read by these sensors are transferred to means 14 and 20.

The block 14 plays a role of selecting several sensors that can be used. This relates to selecting two sensors in the seating portion 10 and two sensors in the seat back 12, for example. However, the number of sensors can be reduced or increased according to the need.

It is assumed that an engine of the vehicle has been stopped. The driver and passenger enter the vehicle. When a switch is turned on by an ignition key or an equivalent member, a device 7 according to the present invention including means 14, 20, 22 instructs the use of the present method. In the device 7, a member for processing and a member for computing are accommodated in, for example, a dashboard 11.

When the use of the present method is started, the device 7 operates a default substrate of a sensor 6 basically selected from sensors of the seat portion and seat back. This substrate has a memory. This default substrate composes part of a basic adjustment according to the present method.

If the body of the human 2 is on the seat 8, several sensors 6 transmit signals. The means 14 analyzes these signals and takes various situations, especially characteristics of the body 2 and its posture on the seat into consideration thereby to estimate whether or not it is preferable to adjust the substrate. The means 14 also analyzes whether or not an object other than a human exists on the member. If the object exists, no additional processing is performed.

For this purpose, the means 14 basically identifies the sensor 6 to supply the most usable signal. A sensor that does not supply any signal is not taken into consideration. A sensor that supplies a high pressure signal is not taken into consideration, either. This is because the present method examines especially a change of pressure. Accordingly, a sensor placed under the buttock of the human 2 receives most weight of the trunk of the human and supplies a signal with relatively poor information. These signals are not generally taken into consideration. In this stage, a substrate most related to the means 14 must be obtained; in this case, a substrate having a sensor that detects a slight movement, that is, a slight change of pressure must be obtained. For this purpose, several sensors are added or removed thereby to make a basic substrate adapt to. In such an adapted substrate, a sensor is selected in the latter stage of the present method.

In the latter stage of the present method, whenever a slow body movement or a movement with a small amplitude is detected, the block 14 is configured to select a new sensor that can be associated with the movement. When a quick body movement or a movement with a large amplitude is detected, the block 14 completely renews the substrate of the sensor thereby to adapt to the situation more suitably.

Next, it will be described how the block selects the most suitable sensor, that is, a sensor that has the highest possibility of including a desired signal associated with a biological parameter in the presence of a predetermined sensor substrate.

a. Selecting Signal

Simultaneous analysis of time - frequency is performed here. Therefore, atomic resolution providing high frequency accuracy is used to estimate a weight, and thereby the position of a component to be tracked can be located. This is kind of extension by wavelet packet.

Therefore, one sensor signal is represented by linear coupling of an extension function $f_{m,n}$.

[Expression 7]

$$x_n = \sum_{m=1}^{M} \alpha_m f_{m,n}$$

This signal x can be represented by a description method of a matrix as follows: x = Fα, where F = [f$_1$,f$_2$,...,f$_M$]

Here, the signal x represents a column vector (N×1 formant); α represents a column vector whose spreading coefficient is (N×1); and F represents a matrix of N×M whose column is an extension function $f_{m,n}$. One linear coupling of the spreading coefficient and various functions supplies one signal model. A plurality of compact models tend to include an extension function having a strong correlation with a signal.

An atom dictionary to adapt to a wide range of time - frequency behavior is prepared, and a signal can be decomposed by selecting several suitable atoms in the atom dictionary. This dictionary is configured as follows.

A pulse reply of a piezoelectric sensor is known to have an attenuation sinusoidal waveform with a basic frequency offset. Therefore, in order to be able to cover all phases, when $g_\gamma$ is a cosine waveform and $h_\gamma$ is a sinusoidal waveform, if the dictionary is composed from a vector D = ($g_\gamma$,hγ) system, the highly suitable dictionary can be obtained.

In this way, the dictionary is composed of a sinusoidal waveform and cosine waveform of various possible frequencies (frequencies limited to a monitoring range according to the present invention). In this case, since the strongest frequency is 20 hertz, it is preferable to use a frequency range between 0.2 hertz to 3 hertz for a single respiratory signal in this embodiment. For two signals obtained by combining a respiratory signal and a heart rhythm, the range between 0.7 to 20 hertz is used. In either case, 1 pitch is set to be 0.1 hertz.

In order to obtain a sufficient frequency resolution, each atom of the dictionary is weighted by a hanning window thereby to avoid, especially, an edge effect.

[Expression 8]

$$\begin{cases} h_\gamma = w.\sin 2\pi\gamma k \\ g_\gamma = w.\cos 2\pi\gamma k \end{cases}$$

where

[Expression 9]

$$w = \frac{1}{2}(1 - \cos(2\pi(1:m)/m+1))$$

where m represents a length of an atom. Practically, this length is an important value for a frequency resolution. If there is no such a weight, atoms may have different durations in the dictionary.

Accordingly, the dictionary is composed ofN pieces of weighted sine atoms and N pieces of weighted cosine atoms. These atoms form a compact ( limited, that is, composed of finite number of points other than zero) support signal that can be equated with a wavelet packet by analogy.

Accordingly, Fig. 4 illustrates one of atoms of the dictionary including coupling of one sine atom and one cosine atom for one frequency. Time of the signal is represented by the axis of abscissas and the amplitude of the signal is represented by the axis of ordinate. In this case, a length of the atom can be measured between the point of 0 samples and the point of 2000 samples on the axis of abscissas, and time is selected as the number of samples (depending on a sampling frequency).

Moreover, a normalized coefficient is calculated for each pair of atoms.

[Expression 10]

$$\begin{cases} \phi_{1,\gamma} = <h_\gamma, g_\gamma> \\ \phi_{2,\gamma} = \dfrac{1}{1 - \phi_{1,\gamma}^2} \end{cases}$$

This enables atoms each intrinsically having a different weight and length to be compared.

In this way, the dictionary composing an orthonormal base can be obtained.

A signal f (that is, its pulse reply) supplied from each of the sensors of the substrate is inputted into each pair of the atom dictionary and calculated in terms of value as follows.

$\sup |C (f, g_\gamma, h_\gamma)|$

where $C (f, g_\gamma, h_\gamma)$ represents a distance function. In this example, the next distance function is selected.

$$C(f,\ g_\gamma,\ h_\gamma) = \varphi_{2,\gamma} \cdot (<f,g_\gamma>^2 + <f,h_\gamma>^2 - 2\varphi_{1,\gamma}<f,g_\gamma> \cdot <f,h_\gamma>)$$

This distance function permits accurately locating a position of a component simultaneously generated by the human body and the system (a sympathetic vibration by a weight of the human body and vibration of the vehicle). Therefore, if a signal transferred by the sensor has both of a parameter of the system and a biological parameter of the human, the sensor is deemed to be eligible. If the signal includes only a parameter of the system, the sensor is not eligible.

In such a case, by classifying sensors in the order of values calculated for a signal from the largest to the smallest, a list of eligible sensors is generated. The number of natural numbers p is previously decided, and the first p pieces of values in this list are taken into consideration. The first p pieces of sensors corresponding to these values are selected sensors.

b. Predicting movement

In the present method, a human body movement can be predicted in order to appropriately maintain a set of related signals (a biological parameter of an occupant). Therefore, in this example, the following approach is used.

The generation of movement of the body is detected by a sensor on a substrate where a supplied signal starts changing. In Fig. 3, it is assumed that sensors on the substrate in the seat back are sensors identified based on their references (i, j), (i+1, j+1) and (i, j+2).

The means 14 identifies the movement with the use of these sensors, and also can predict a direction of the movement indicated by an arrow 24 in Fig. 3 by interpolation. Therefore, during such a movement, the means 14 predicts a future movement, and during the movement, adds a sensor to the substrate on which the selected sensor is placed, the added sensor being placed on the course of the movement and being able to supply an advantageous signal during the future movement. In Fig. 3, two sensors (i+2, j+2), (i+1, j+2) placed on the same row of a sensor (i, j+2) are added sensors. Accordingly, the means 14 can take signals to be supplied by these sensors into consideration as soon as possible. After that, if it is confirmed that movement can be recognized at positions of these sensors, these sensors remain placed on the substrate.

On the contrary, if the prediction is an error and no movement can be detected by at least one of the sensors, this sensor is removed from the substrate.

2. Identification and Estimation of Transfer Function

A plurality of accelerometers 16 are used to play a role as references for vibration noise in the vehicle. Unless the vibration in the vehicle is exerted in a single direction, for example, in only a vertical direction, it is preferable to use a triaxial accelerometer or 3D accelerometer. Moreover, it is preferable to use at least two accelerometers.

In some cases, it is determined that it is important to specify positions of these accelerometers in order to obtain a highly reliable model. For example, by placing one accelerometer 16 in a lower portion of a structure of the seating portion 10 composing the seat, this accelerometer is adapted to detect vibration of the occupant under the seat. In this embodiment, the second accelerometer is placed on the top of the seat back 12. This is because it is confirmed that this portion of the seat can vibrate independently, to some extent, from the seating portion.

According to position determination of the piezoelectric sensors 6 and accelerometers 16, modeling a transfer function, which is performed after the position determination, can be linear or non-linear. In either case, a parameter for such modeling is estimated by a recursive procedure. Moreover, the parameter is often re-estimated during performing the present method so that the model accurately adapts to various situations, especially to driving conditions.

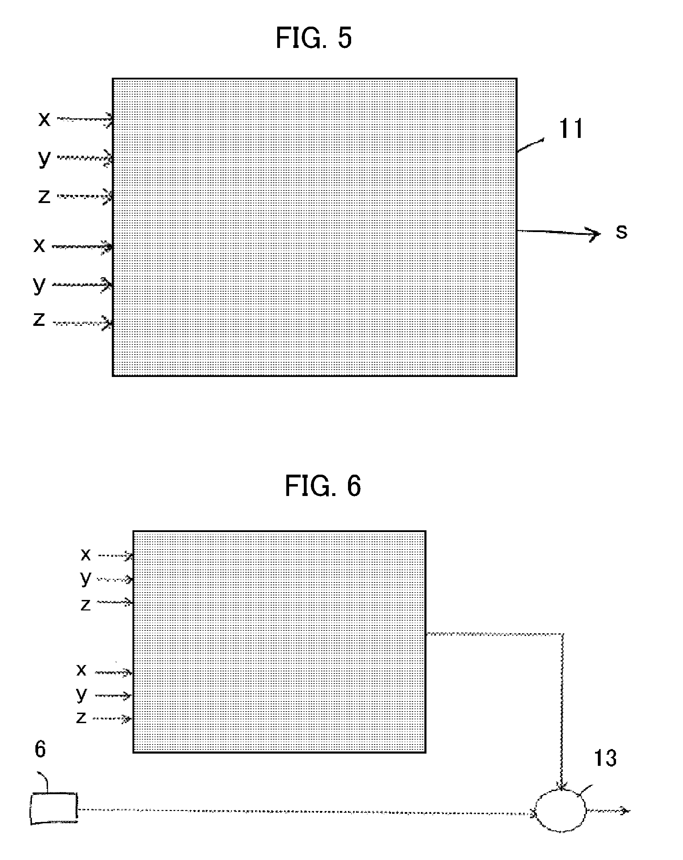

A transfer function is modeled for each of the selected piezoelectric sensors 6. Accordingly, as illustrated in Fig. 5, this transfer function has output signals from all accelerometers 16 in input. In this case, the output signals are three signals supplied by accelerometers 16 in the seating portion, each corresponding to each of vibrations in the direction of X, Y and Z, as well as three similar signals supplied by accelerometers 16 in the seat back. A transfer function has a signal s supplied by the piezoelectric sensor 6 that is taken into consideration in output. Fig. 5 illustrates a principle for modeling the transfer function. This relates to identifying a function 11 and its parameter. Input has x, y, z signals supplied by two accelerometers, and output has the signal s that is supplied by the piezoelectric sensor taken into consideration. In this way, an effect unique to vibration in a signal supplied by the piezoelectric sensor can be modeled.

In this case, the means 20 first decides a model that is most suitable for obtaining a transfer function from a list of a plurality of types of models, according to a situation. The list is as follows, in this case.
Modeling by indicating a state.
ARMA,
ARX,

NLARX.

After attempting the modelling based on each type of model, the most suitable model is basically taken into consideration.

Next, as illustrated in Fig. 6, with the use of the model identified in this way, a noise value is dynamically decided according to momentary signals supplied by the accelerometers. Inputted are six signal values from the accelerometer. Outputted is an estimation value by a single noise of the signal side of the piezoelectric sensor. In this case, this estimation value is subtracted from a signal supplied by the piezoelectric sensor 6 at the position of a subtractor 13. After this subtraction, a signal of which most of vibration noise effect is removed can be obtained.

In the present embodiment, the means 20 uses an ARX-type exogenous autoregressive model by default. This means that if a better result cannot be obtained from any of the other types of models in the list, this type of model is used. Otherwise, a model to provide the best result is used. The structure of this model is as follows.

[Expression 11]

$$A(q) \cdot y(t) = \sum_{1}^{Ni} B_i(q) \cdot u_i(t - n_{ki}) + e(t)$$

where

$A(q)$ represents a polynomial expression having $N_A$ pieces of coefficients.

$y(t)$ represents an output signal of the piezoelectric sensor.

$B_i(q)$ represents a polynomial expression having $N_B$ pieces of coefficients.

$u_i(t)(i = 1...N_i)$ represents an input signal supplied by the accelerometer.

$n_{ki}$ represents the number of unit delay in input.

$e(t)$ represents an error signal in this model.

The total number of free coefficients $N_C$ can be found as follows.

$$N_C = N_A + N_i \cdot N_B$$

A coefficient of a polynomial expression is estimated by minimizing a trace of an error prediction covariance matrix. As described above, such estimation of a parameter is sometimes updated according to a change of driving conditions. When a parameter of a model is estimated at each sampling step, prediction noise (that is, estimation noise) in the piezoelectric sensor can be calculated. In this case, such estimation noise is removed in output from the piezoelectric sensor, as illustrated in Fig. 6.

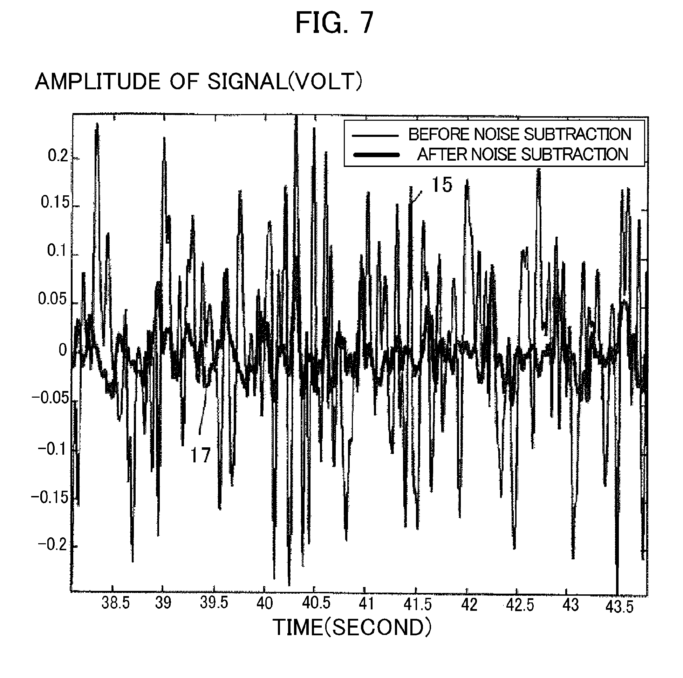

In Fig. 7, a signal 15 of the piezoelectric sensor 6 before the subtraction is indicated by a thin line, and the signal 17 after the subtraction is indicated by a thick line. Especially, an amplitude (unit: volt) of the signal on the ordinate extremely decreases after the subtraction, which shows that peaks corresponding to heartbeats become apparent.

3. Extraction of Biological Parameter

After such a noise removing step, the means 20 should extract a heart rhythm and a respiratory signal. This attributes to estimating a parameter whose frequency cannot be directly observed. Accordingly, it is especially effective to use Bayesian estimation. Moreover, since a studied system is non-linear, the extended Kalman filter can be used. In order to closely recognize a change of noise other than gaussian noise, the individual filter may be used.

By way of example, using the extended Kalman filter to estimate and monitor a heartbeat will be described below.

It is suggested that a piezoelectric sensor signal in response to a blood pressure is modeled by adding higher harmonic wave components of a sinusoidal waveform having a slowly-changing amplitude element and phase element.

[Expression 12]

$$y(t) = \sum_{i=1}^{m} a_i(t) \cdot \sin \phi_i(t)$$

where $\varphi_1(t) = \omega(t) \cdot t$,

$\varphi_i(t) = i \cdot \omega(t) \cdot t + \theta_i(t)$, $i = 2 \ldots m$,

$\omega(t)$ represents a basic pulse of a signal associated with a heartbeat. m represents the number of sinusoidal waveform components,

$a_i(t)$ represents an amplitude of sinusoidal waveform component,

$\varphi_i(t)$, $i = 2 \ldots m$ represents a momentary phase of a higher harmonic wave, and

$\theta_i(t)$ represents a phase difference between the basic pulse and higher harmonic wave.

From this expression, the vector indicating the following state is suggested.

[Expression 13]

$$\hat{\mathbf{x}}_k = \begin{bmatrix} \omega_k \\ a_{k,1} \\ \vdots \\ a_{k,m} \\ \phi_{k,1} \\ \vdots \\ \phi_{k,m} \end{bmatrix}$$

A change of an amplitude $a_{k,l}$ of the sine component over time is modeled by additional white gaussian noise as follows.

$$a_{k+1,i} = a_{k,i} + v_{k,i}{}^{a}$$

A change over time of a momentary basic pulse $\omega_k$ is also modeled by additional white gaussian noise.

$$\omega_{k+1} = \omega_k + v_k{}^{\omega}$$

A phase difference $\theta_i(t)$ between a basic pulse and a higher harmonic wave component is similar to the above, and a change over time of a momentary phase $\varphi_{k,i}(t)$ can be obtained by the following expression.

$$\varphi_{k+1,i} = i \cdot \omega_k + \varphi_{k,i} + v_{k,i}{}^{\varphi}$$

Such a selection means that $\omega_k$ is represented by a ratio between a real pulse and a sampling frequency of a signal. As a result, an expression representing a state transition is linear and given by the following expression.

[Expression 14]

$$\mathbf{x}_{k+1} = \mathbf{F}(\mathbf{x}_k, \mathbf{v}_k) = \begin{bmatrix} \omega_k \\ a_{k,1} \\ \vdots \\ a_{k,m} \\ 1 \cdot \omega_k + \phi_{k,1} \\ \vdots \\ m \cdot \omega_k + \phi_{k,m} \end{bmatrix} + \mathbf{v}_k$$

The above expression can also be written as follows.

$$x_{k+1} = A \cdot x_k + v_k \quad (1)$$

where

[Expression 15]

$$\mathbf{A} = \begin{bmatrix} 1 & & & & & & \\ & 1 & & & & & \\ & & \ddots & & & & \\ & & & 1 & & & \\ 1 & & & & 1 & & \\ \vdots & & & & & \ddots & \\ m & & & & & & 1 \end{bmatrix} \quad (2)$$

Estimating a variance of components of noise $v_k$ has an effect on a speed of change of an estimated parameter (pulse, amplitude component and phase component) and a convergence speed of algorithm.

Taking Expression (1) into consideration, an expression representing predicted observation is basically given by the following expression.

[Expression 16]

$$\hat{\mathbf{y}}_k^- = \mathbf{H}(\hat{\mathbf{x}}_k^-, \mathbf{w}_k) = \sum_{i=1}^{m} \hat{a}_{i,k} \cdot \sin \hat{\phi}_{i,k} + \mathbf{n}_k \quad (3)$$

The expression representing observation is non-linear, which supports the use of the extended Kalman filter. A variance of observed noise $n_k$ is associated with a noise variance observed in a piezoelectric signal.

The algorithm of the extended Kalman filter is executed as follows.

Initialization Step:

[Expression 17]

$$\hat{\mathbf{x}}_0 = \mathrm{E}\left[\mathbf{x}_0\right]$$

$$\mathbf{P}_{\mathbf{x}_0} = \mathrm{E}\left[\left(\mathbf{x}_0 - \hat{\mathbf{x}}_0\right) \cdot \left(\mathbf{x}_0 - \hat{\mathbf{x}}_0\right)^T\right]$$

In the case of $k \in \{1,...,\infty\}$, a prediction expression of the extended Kalman filter is as follows.

[Expression 18]

$$\hat{\mathbf{x}}_k^- = \mathbf{F}(\hat{\mathbf{x}}_{k-1}, \overline{\mathbf{v}})$$

$$\mathbf{P}_{\mathbf{x}_k}^- = \mathbf{A}_{k-1} \cdot \mathbf{P}_{\mathbf{x}_{k-1}} \cdot \mathbf{A}_{k-1}^T + \mathbf{W}_k \cdot \mathbf{Q}^{\mathbf{w}} \cdot \mathbf{W}_k^T$$

An updated expression is as follows.

[Expression 19]

$$\mathrm{K}_k = \mathbf{P}_{\mathbf{x}_k}^- \cdot \mathbf{C}_k^T \cdot \left(\mathbf{C}_k \cdot \mathbf{P}_{\mathbf{x}_{k-1}}^- \cdot \mathbf{C}_k^T + \mathbf{V}_k \cdot \mathbf{R}^v \cdot \mathbf{V}_k^T\right)^{-1}$$

$$\hat{\mathbf{x}}_k = \hat{\mathbf{x}}_k^- + \mathrm{K}_k \cdot \left(\mathbf{y}_k - \mathbf{H}(\hat{\mathbf{x}}_k^-, \mathbf{w}_k)\right)$$

$$\mathbf{P}_{\mathbf{x}_k} = \mathbf{C}_k^T \cdot \left(\mathbf{I} - \mathrm{K}_k \cdot \mathbf{C}_k\right) \cdot \mathbf{P}_{\mathbf{x}_k}^-$$

where

[Expression 20]

$$A_k \overset{\Delta}{=} \frac{\partial F(x, \overline{v})}{\partial x}\Bigg|_{\hat{x}_k} \; , \; W_k \overset{\Delta}{=} \frac{\partial F(\hat{x}_k^-, v)}{\partial v}\Bigg|_{\overline{v}} \; ,$$

$$C_k \overset{\Delta}{=} \frac{\partial H(x, \overline{n})}{\partial x}\Bigg|_{\hat{x}_k} \; , \; V_k \overset{\Delta}{=} \frac{\partial H(\hat{x}_k^-, n)}{\partial n}\Bigg|_{\overline{n}}$$

where $Q^w$ and $R^\eta$ are represent covariance matrixes of $v_k$ and $n_k$, respectively; and I represents a unit matrix.

Accordingly, this is a standard algorithm relating to the extended Kalman filter.

According to the present invention, this algorithm is applied as follows. It is assumed that the values of noises

[Expression 21]

$$\overline{v} = E\begin{bmatrix} v \end{bmatrix}$$

and

[Expression 22]

$$\overline{n} = E\begin{bmatrix} n \end{bmatrix}$$

are equal to zero.

Since it is known that Expression (1) representing a state transition is linear, the following expression can be obtained.

[Expression 23]

$$\mathbf{A}_k \stackrel{\Delta}{=} \left.\frac{\partial \mathbf{F}(\mathbf{x}, \overline{\mathbf{v}})}{\partial \mathbf{x}}\right|_{\hat{\mathbf{x}}_k} = \mathbf{A} \quad (4)$$

where a can be obtained in Expression (2).

**[0095]** Taking Expressions (1) and (3) into consideration, the following expression can be obtained.

[Expression 24]

$$\mathbf{W}_k \stackrel{\Delta}{=} \left.\frac{\partial \mathbf{F}(\hat{\mathbf{x}}_k^-, \mathbf{v})}{\partial \mathbf{v}}\right|_{\overline{\mathbf{v}}} = \mathbf{I}^{2 \cdot m+1}$$

and

[Expression 25]

$$\mathbf{V}_k \stackrel{\Delta}{=} \left.\frac{\partial \mathbf{H}(\hat{\mathbf{x}}_k^-, \mathbf{n})}{\partial \mathbf{n}}\right|_{\overline{\mathbf{n}}} = 1 \quad (5)$$

**[0096]** Finally, Expression (3) is as follows.

[Expression 26]

$$\mathbf{C}_k \overset{\Delta}{=} \frac{\partial \mathbf{H}(\mathbf{x}, \overline{\mathbf{n}})}{\partial \mathbf{x}}\bigg|_{\hat{\mathbf{x}}_k} = \Big[ 0 \quad \sin \hat{\overline{\phi}}_{k,1} \quad \ldots \quad \sin \hat{\overline{\phi}}_{k,m}$$

$$\hat{\overline{a}}_{k,1} \cdot \cos \hat{\overline{\phi}}_{k,1} \quad \ldots \quad \hat{\overline{a}}_{k,m} \cdot \cos \hat{\overline{\phi}}_{k,m} \Big] \quad (6)$$

In order to simultaneously manipulate a plurality of signals from the piezoelectric sensors, lengths of the state vector and observation vector can also be lengthened. For example, by using two signals from the sensors, the state vector, state transition matrix, and linear matrix representing observation are as follows.

[Expression 27]

$$\hat{\mathbf{x}}_k = \begin{bmatrix} \omega_k \\ a_{k,1,1} \\ \vdots \\ a_{k,1,m} \\ a_{k,2,1} \\ \vdots \\ a_{k,2,m} \\ \phi_{k,1,1} \\ \vdots \\ \phi_{k,1,m} \\ \phi_{k,2,1} \\ \vdots \\ \phi_{k,2,m} \end{bmatrix}, \quad \mathbf{A} = \begin{bmatrix} 1 & & & & & & & & & \\ & 1 & & & & & & & & \\ & & \ddots & & & & & & & \\ & & & 1 & & & & & & \\ & & & & 1 & & & & & \\ & & & & & \ddots & & & & \\ & & & & & & 1 & & & \\ 1 & & & & & & & 1 & & \\ \vdots & & & & & & & & \ddots & \\ m & & & & & & & & & 1 \\ 1 & & & & & & & & & & 1 \\ \vdots & & & & & & & & & & & \ddots \\ m & & & & & & & & & & & & 1 \end{bmatrix},$$

$$\mathbf{C}_k = \begin{bmatrix} 0 & & & & & 0 \\ \sin \hat{\phi}_{k,1,1}^{-} & & & & & \\ \sin \hat{\phi}_{k,1,m}^{-} & & & & & \\ \hat{a}_{k,1,1}^{-} \cdot \cos \hat{\phi}_{k,1,1}^{-} & & & & & \\ \hat{a}_{k,1,m}^{-} \cdot \cos \hat{\phi}_{k,1,m}^{-} & & & & & \\ & & \sin \hat{\phi}_{k,2,1}^{-} & & & \\ & & \sin \hat{\phi}_{k,2,m}^{-} & & & \\ & & \hat{a}_{k,2,1}^{-} \cdot \cos \hat{\phi}_{k,2,1}^{-} & & & \\ & & \hat{a}_{k,2,m}^{-} \cdot \cos \hat{\phi}_{k,2,m}^{-} \end{bmatrix}^{T} \qquad (7)$$

In this processing, in order to find a heartbeat (and/or a respiratory signal), one or more piezoelectric sensors can be processed. This model representing a state is cited only as an example, but not restrictive.

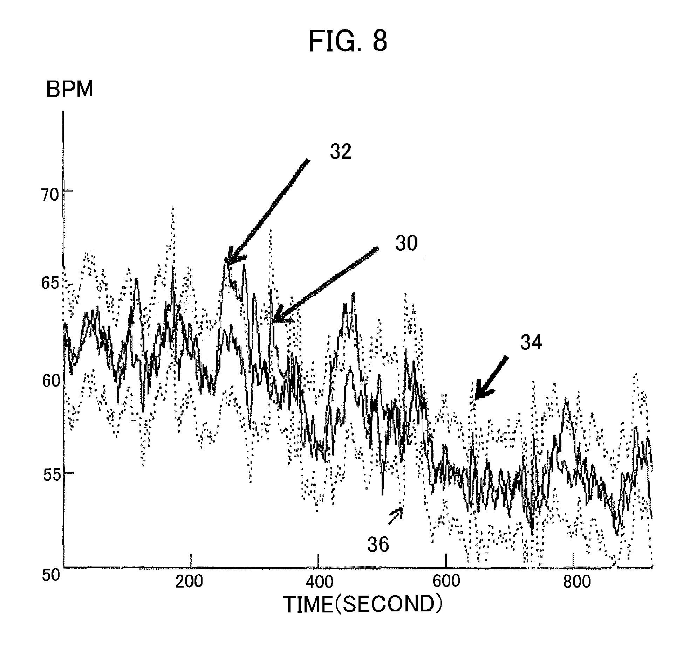

A result of the above processing is illustrated by way of example in Fig. 8. A curve 30 represents a real heartbeat signal; a curve 32 represents a heartbeat signal estimated by a method according to the present invention; and curves 34 and 36 represent tolerance thresholds of plus or minus 5% of an amplitude of the real signal. These curves indicate the number of pulses per minute on the ordinate corresponding to time (unit: second) indicated on the abscissa. The graph shows that most of differences between the real signal and the signal estimated by a method according to the present invention are included in the tolerance range of plus or minus 5% of the real signal.

This relates to a result of a test performed under real driving conditions (for example, driving in cities and driving on an expressway).

The means 14, 20, 22 include a calculation means such as a microprocessor and, one or more computers with, for example, one or more memories. A method according to the present invention can be automatically performed by a computer program recorded on a data recording medium such as a hard disk, flash memory, CD or DVD. The computer program includes a code instruction that can control performance of a method of the present invention when the method is executed by the computer. Such a program can be configured to be used in a remote communication network for downloading, for example, downloading an updated program version.

It should be appreciated that various modifications can be made to the present invention without departing from the scope of the present invention.

An example has been described in which selection step 1, noise removing step 2 with the use of a transfer function, and non-linear filtering step 3 are consecutively performed. As supported by Fig. 8, this consecution produces a highly desirable result. However, in the environment with less noise, only any one of these steps or any two of these steps can also be used with obtaining an acceptable result.

In a first step, a unique atom dictionary can be associated with each of frequency ranges, and accordingly, in this case, two atom dictionaries can be used.

The present application is based on French Patent Application No. 0951714 filed on March 18, 2009. The entire specification, claims and drawings of French Patent Application No. 0951714 are incorporated herein by reference.

**Claims**

1. A method for monitoring at least one biological parameter out of a heartbeat and/or a respiratory signal of an occupant on a member of a seat or a bed, the method **characterized by** comprising the step of:

    processing a signal or respective signals by non-linear filtering, the signal or respective signals being received from one or more sensors that is/are connected to the member and can detect a change of pressure due to contact.

2. The method according to claim 1, wherein the filtering includes a Bayesian recursive estimator such as an extended Kalman filter or an individual filter.

3. The method according to claim 1 or 2, wherein a linear state transition expression of a following type is used:

$$x_{k+1} = A \cdot x_k + v_k \, ,$$

    where $x_{k+1}$ is a vector representing a state of a following type,

[Expression 1]

$$\hat{\mathbf{x}}_k = \begin{bmatrix} \omega_k \\ a_{k,1} \\ \vdots \\ a_{k,m} \\ \phi_{k,1} \\ \vdots \\ \phi_{k,m} \end{bmatrix}, \quad \mathbf{A} = \begin{bmatrix} 1 & & & & & & \\ & 1 & & & & & \\ & & \ddots & & & & \\ & & & 1 & & & \\ 1 & & & & 1 & & \\ \vdots & & & & & \ddots & \\ m & & & & & & 1 \end{bmatrix}$$

and $v_k$ is white gaussian noise.

4. The method according to any one of claims 1 to 3, wherein an output signal from the sensor or one of the pluirality of sensors is modeled according to a following expression,

[Expression 2]

$$y(t) = \sum_{i=1}^{m} a_i(t) \cdot \sin \phi_i(t)$$

where
$\varphi_1(t) = \omega(t) \cdot t$
$\varphi_i(t) = i \cdot \omega(t) \cdot t + \theta_i(t)$, i = 2 ... m,
y(t) represents a signal,
$\omega(t)$ represents a momentary basic pulse of the signal,
m represents the number of sine functions,
$a_i(t)$ represents an amplitude of a sine function,
$\varphi_i(t)$ represents a momentary phase of a higher harmonic wave, and
$\theta_i(t)$ represents a phase difference between the basic pulse and the higher harmonic wave.

5. The method according to any one of claims 1 to 4, wherein an observation expression of a following type is used,

[Expression 3]

$$\hat{\mathbf{y}}_k^- = \mathbf{H}(\hat{\mathbf{x}}_k^-, \mathbf{w}_k) = \sum_{i=1}^{m} \hat{a}_{i,k} \cdot \sin \hat{\phi}_{i,k} + \mathbf{n}_k$$

where

[Expression 4]

$$\hat{\mathbf{y}}_k^-$$

represents an estimation value of a signal y(k) by an observer,
H represents a matrix associating a state $x_k$ with a measured value $y_k$,

[Expression 5]

$$\hat{a}_{i,k}$$

and

[Expression 6]

$$\hat{\phi}_{i,k}$$

represent estimation values of $a_{i,k}$ and $\varphi_{i,k}$ by the observer, respectively, and
$n_k$ represents noise observed by the observer.

6.   The method according to any one of claims 1 to 5, comprising the steps of:

    receiving a signal from each of a group of sensors,
    inputting this signal to an atom dictionary,
    selecting several sensors on a basis of the input, and
    using only the selected sensors to perform monitoring.

7. The method according to any one of claims 1 to 6, comprising the steps of:

connecting at least one accelerometer to the member,
deciding a model for a transfer function between at least one signal in input from the accelerometer or one of the at least one of the accelerometers and a signal in output from the sensor or one of the plurality of sensors,
estimating a noise value with use of the model, and
removing the estimated noise value from the signal of the sensor.

8. The method according to any one of claims 1 to 7, the method being mounted on a vehicle and used.

9. A computer program **characterized by** including a code instruction that can control performance of the steps of a method according to any one of claims 1 to 8 when the method is performed in a computer or a calculator.

10. A device for monitoring at least one biological parameter out of a heartbeat and/or a respiratory signal of an occupant on a member of a seat or a bed, the device **characterized by** comprising:

at least one sensor that is connected to the member and can detect a change of pressure due to contact; and
a unit to process a signal or respective signals by non-linear filtering, the signal or respective signals being received from the sensor or the respective sensors.

# FIG. 1

# FIG. 2

# FIG. 3

# FIG. 4

AMPLITUDE

TIME(SAMPLING)

FIG. 5

FIG. 6

# FIG. 7

AMPLITUDE OF SIGNAL(VOLT)

FIG. 8

## INTERNATIONAL SEARCH REPORT

| International application No. |
|---|
| PCT/JP2010/054701 |

A.  CLASSIFICATION OF SUBJECT MATTER
*A61B5/00*(2006.01)i, *A61B5/11*(2006.01)i

According to International Patent Classification (IPC) or to both national classification and IPC

B.  FIELDS SEARCHED

Minimum documentation searched (classification system followed by classification symbols)
A61B5/00, A61B5/11

Documentation searched other than minimum documentation to the extent that such documents are included in the fields searched
| | | | |
|---|---|---|---|
| Jitsuyo Shinan Koho | 1922–1996 | Jitsuyo Shinan Toroku Koho | 1996–2010 |
| Kokai Jitsuyo Shinan Koho | 1971–2010 | Toroku Jitsuyo Shinan Koho | 1994–2010 |

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
|---|---|---|
| Y<br>A | JP 2008-113797 A  (Aisin Seiki Co., Ltd.),<br>22 May 2008 (22.05.2008),<br>fig. 1, 2<br>(Family: none) | 1,2,8-10<br>3-7 |
| Y | WO 2007/143535 A2  (BIANCAMED LTD.),<br>13 December 2007 (13.12.2007),<br>paragraph [0044]<br>& JP 2009-538720 A      & US 2009/0203972 A1<br>& EP 2020919 A | 1,2,8-10 |
| Y | JP 2005-161039 A  (Siemens Medical Solutions<br>Health Services Corp.),<br>23 June 2005 (23.06.2005),<br>paragraph [0072]<br>& US 2005/0119866 A1     & GB 2408124 A<br>& DE 102004054319 A | 2 |

☒  Further documents are listed in the continuation of Box C.          ☐  See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>    24 May, 2010 (24.05.10) | Date of mailing of the international search report<br>    01 June, 2010 (01.06.10) |
|---|---|
| Name and mailing address of the ISA/<br>    Japanese Patent Office | Authorized officer |
| Facsimile No. | Telephone No. |

Form PCT/ISA/210 (second sheet) (July 2009)

**INTERNATIONAL SEARCH REPORT**

| International application No. |
| --- |
| PCT/JP2010/054701 |

C (Continuation).    DOCUMENTS CONSIDERED TO BE RELEVANT

| Category* | Citation of document, with indication, where appropriate, of the relevant passages | Relevant to claim No. |
| --- | --- | --- |
| Y | JP 2008-79931 A  (Aisin Seiki Co., Ltd.),<br>10 April 2008 (10.04.2008),<br>paragraph [0002]<br>& US 2010/0056937 A     & WO 2008/038501 A1<br>& DE 112007002169 T | 8 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20080103702 A
- FR 0951714